# EUROPEAN PATENT APPLICATION

(11) **EP 4 810 045 A1**
(43) Date of publication of application: **23.09.2026**
(21) Application number: 24890642.2
(22) Date of filing: 12.11.2024
(51) Int. Cl.: A61K 31/5517, A61K 9/20, A61P 35/00, A61K 35/04

(54) **PHARMACEUTICAL COMPOSITION AND USE THEREOF**

(30) Priority: 13.11.2023 CN 202311508354
(71) Applicant: TransThera Sciences (Nanjing), Inc., Nanjing, Jiangsu 210032 (CN)
(72) Inventor: TONG, Qingchen, Nanjing, Jiangsu 210032 (CN)
(74) Representative: Winter, Brandl - Partnerschaft mbB
(86) International application number: PCT/CN2024/131446
(87) International publication number: WO 2025/103287

(57) **Abstract**

The present invention belongs to the technical field of medicines, and particularly relates to a pharmaceutical composition and the use thereof. The pharmaceutical composition comprises 1wt%-70wt% of a compound of formula (I) or a pharmaceutically acceptable salt, crystal form and isomer thereof, and at least one pharmaceutical adjuvant. By means of research, the present invention develops the pharmaceutical composition which can be prepared into final dosage forms, such as tablets, capsules and particles. When the pharmaceutical composition is prepared into a tablet, the pharmaceutical composition has good compressibility, good dissolution rate and good particle fluidity when compared to other pharmaceutical combination modes.

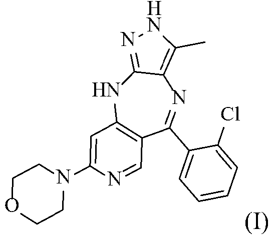

## Description

### TECHNICAL FIELD

The present disclosure pertains to the technical field of medicines, and particularly relates to a pharmaceutical composition and use thereof.

### BACKGROUND

WO2018108079A1 discloses a compound represented by formula (I). The compound is a novel mechanism-based kinase spectrum-selective focused multi-target small molecule kinase inhibitor, which can act on multiple targets including FGFR1-3, KDR, Aurora A/B, JAK, and the like, demonstrating a good anti-tumor effect.

Currently, there is no literature reporting on the formulation studies of the compound. Therefore, to meet clinical needs, the inventors still need to investigate the physical and chemical characteristics of the active pharmaceutical ingredient, aiming to obtain a stable and pharmaceutically acceptable pharmaceutical formulation.

### SUMMARY

The present disclosure investigates a pharmaceutical composition comprising 1 wt%-70 wt% of an active ingredient, wherein the active ingredient is a compound of formula (I) or a pharmaceutically acceptable salt, crystal form, or isomer thereof, and at least one pharmaceutical excipient;

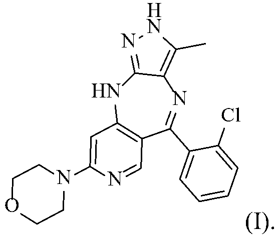

In some embodiments, in the pharmaceutical composition described in the present disclosure, the pharmaceutical excipient comprises a filler, a binder, a disintegrant, or a lubricant.

In some embodiments, in the pharmaceutical composition described in the present disclosure, the pharmaceutical excipient further comprises a glidant.

In some embodiments, the pharmaceutical composition described in the present disclosure comprises 1 wt%-30 wt% of the compound of formula (I) or the pharmaceutically acceptable salt, crystal form, or isomer thereof; 10 wt%-90 wt% of the filler; 0.1 wt%-10 wt% of the binder; 1 wt%-10 wt% of the disintegrant; and 0.1 wt%-2 wt% of the lubricant.

In some embodiments, the pharmaceutical composition described in the present disclosure further comprises 0.1 wt%-2 wt% of the glidant.

Preferably, the pharmaceutical composition described in the present disclosure comprises 1 wt%-20 wt% of the compound of formula (I) or the pharmaceutically acceptable salt, crystal form, or isomer thereof; 80 wt%-90 wt% of the filler; 2 wt%-5 wt% of the binder; 2 wt%-8 wt% of the disintegrant; and 0.6 wt%-1 wt% of the lubricant.

Preferably, the pharmaceutical composition described in the present disclosure comprises 1 wt%-10 wt% of the compound of formula (I) or the pharmaceutically acceptable salt, crystal form, or isomer thereof; 80 wt%-90 wt% of the filler; 2 wt%-5 wt% of the binder; 2 wt%-8 wt% of the disintegrant; and 0.6 wt%-1 wt% of the lubricant.

Preferably, the pharmaceutical composition described in the present disclosure further comprises 0.5 wt%-1 wt% of the glidant.

In some embodiments, the pharmaceutical composition of the present disclosure comprises 1 wt%-10 wt% of the compound of formula (I) or the pharmaceutically acceptable salt, crystal form, or isomer thereof.

Preferably, the pharmaceutical composition of the present disclosure comprises 1 wt%-5 wt%, 2 wt%-5 wt%, or 2.5 wt%-5 wt% of the compound of formula (I) or the pharmaceutically acceptable salt, crystal form, or isomer thereof.

In some embodiments, in the pharmaceutical composition of the present disclosure, the filler is selected from: one or more of lactose monohydrate, microcrystalline cellulose, corn starch, glucose, mannitol, sorbitol, calcium carbonate, and calcium hydrogen phosphate.

Preferably, the filler is selected from lactose monohydrate and microcrystalline cellulose.

Preferably, the model of the lactose monohydrate is lactose monohydrate 200M.

Preferably, the model of the microcrystalline cellulose is microcrystalline cellulose 101.

In some embodiments, the mass ratio of the lactose monohydrate to the microcrystalline cellulose is 1:(0.1-5).

Preferably, the mass ratio of the lactose monohydrate to the microcrystalline cellulose is 1:(0.2-2).

More preferably, the mass ratio of the lactose monohydrate to the microcrystalline cellulose is 1:0.2.

In some embodiments, in the pharmaceutical composition of the present disclosure, the binder is selected from: one or more of povidone, hydroxypropyl cellulose, methyl cellulose, hydroxypropyl methylcellulose, and sodium carboxymethyl cellulose.

Preferably, the binder is selected from povidone.

Preferably, the model of the povidone is povidone K30 or povidone K90.

Preferably, the model of the hydroxypropyl cellulose is hydroxypropyl cellulose EXF.

In some embodiments, in the pharmaceutical composition of the present disclosure, the disintegrant is selected from: one or more of sodium carboxymethyl starch, low-substituted hydroxypropyl cellulose, crospovidone, and croscarmellose sodium; preferably, the disintegrant is selected from sodium carboxymethyl starch.

Preferably, the model of the crospovidone is crospovidone XL-10.

In some embodiments, in the pharmaceutical composition of the present disclosure, the lubricant is selected from: magnesium stearate and sodium stearyl fumarate.

In some embodiments, in the pharmaceutical composition of the present disclosure, the glidant is selected from: colloidal silicon dioxide and talcum powder.

In some embodiments, in the pharmaceutical composition of the present disclosure, a wetting agent may be added according to actual circumstances: water.

In some embodiments, in the pharmaceutical composition described in the present disclosure, the particle size distribution range of the compound of formula (I) or the pharmaceutically acceptable salt, crystal form, or isomer thereof is D90 < 60 µm.

In some embodiments, the particle size distribution range of the compound of formula (I) or the pharmaceutically acceptable salt, crystal form, or isomer thereof is D90 < 59.7 µm.

In some embodiments, the particle size distribution range of the compound of formula (I) or the pharmaceutically acceptable salt, crystal form, or isomer thereof is D90 ≤ 50.4 µm.

In some embodiments, the particle size distribution range of the compound of formula (I) or the pharmaceutically acceptable salt, crystal form, or isomer thereof is D90 ≤ 50 µm.

In some embodiments, in the pharmaceutical composition described in the present disclosure, the particle size distribution range of the compound of formula (I) or the pharmaceutically acceptable salt, crystal form, or isomer thereof is D90 ≤ 59 µm, for example, D90 ≤ 58 µm, D90 ≤ 57 µm, D90 ≤ 56 µm, D90 ≤ 55 µm, D90 ≤ 54 µm, D90 ≤ 53 µm, D90 ≤ 52 µm, or D90 ≤ 51 µm; including but not limited to: 0 µm < D90 ≤ 59 µm; 18 µm ≤ D90 ≤ 55 µm; 18.1 µm ≤ D90 ≤ 55 µm; 18 µm ≤ D90 ≤ 51 µm; and 18.1 µm ≤ D90 ≤ 51 µm.

In some embodiments, in the pharmaceutical composition described in the present disclosure, the particle size distribution range of the compound of formula (I) or the pharmaceutically acceptable salt, crystal form, or isomer thereof is 10 µm < D90 < 60 µm.

In some embodiments, the particle size distribution range of the compound of formula (I) or the pharmaceutically acceptable salt, crystal form, or isomer thereof is 18.1 µm ≤ D90 < 59.7 µm.

In some embodiments, the particle size distribution range of the compound of formula (I) or the pharmaceutically acceptable salt, crystal form, or isomer thereof is 18 µm ≤ D90 < 60 µm.

In some embodiments, the particle size distribution range of the compound of formula (I) or the pharmaceutically acceptable salt, crystal form, or isomer thereof is 18.1 µm ≤ D90 ≤ 50.4 µm.

In some embodiments, the particle size distribution range of the compound of formula (I) or the pharmaceutically acceptable salt, crystal form, or isomer thereof is 18 µm ≤ D90 ≤ 50 µm.

In some embodiments, the particle size distribution range of the compound of formula (I) or the pharmaceutically acceptable salt, crystal form, or isomer thereof is 20 µm ≤ D90 ≤ 50 µm.

In some embodiments, in the pharmaceutical composition described in the present disclosure, the content of the compound of formula (I) or the pharmaceutically acceptable salt, crystal form, or isomer thereof is 1-20 mg. 1-20 mg includes any value between 1 and 20, e.g., 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, or 20 mg.

In some embodiments, the present disclosure further provides a pharmaceutical formulation, which is prepared from the pharmaceutical composition according to any one of the above.

In some embodiments, the pharmaceutical formulation provided by the present disclosure is a tablet.

In some embodiments, the present disclosure further provides a kit comprising the pharmaceutical formulation and instructions for use.

In some embodiments, the present disclosure further provides use of the pharmaceutical composition according to any one of the above or the pharmaceutical formulation in the manufacture of a medicament for treating a cancer.

In some embodiments, the cancer includes cholangiocarcinoma, head and neck cancer, liver cancer, colorectal cancer, osteosarcoma, bladder cancer, prostate cancer, gastric cancer, urothelial carcinoma, lung cancer, endometrial cancer, breast cancer, thyroid cancer, ovarian cancer, peritoneal cancer, renal cancer, B-cell malignancy, oral cancer, and nasopharyngeal carcinoma.

The pharmaceutical composition or formulation of the present disclosure is prepared by a wet granulation method.

In some embodiments, the wet granulation step includes:
pre-mixing a filler with a disintegrant and the active ingredient, and then subjecting the mixture to wet granulation with a binder, followed by drying to obtain dry granules.

In some embodiments, after the wet granulation step, the method further includes a final mixing step, i.e., mixing the obtained dry granules with a lubricant and a glidant.

The "D90" described in the present disclosure refers to the particle size value corresponding to 90% of the cumulative particle size distribution of a sample.

The "wt%" described in the present disclosure refers to a mass (weight) percentage.

### BRIEF DESCRIPTION OF THE DRAWINGS

FIG. 1 is an exemplary diagram showing tablets with a smooth and intact surface and good compressibility in the compressibility evaluation;
FIG. 2 is an exemplary diagram showing tablets with sticking issues in the compressibility evaluation.

### Beneficial Effects of the Present Disclosure

By means of research, the present disclosure develops a pharmaceutical composition, which can be prepared into final formulations, such as tablets, capsules, and granules. When the pharmaceutical composition is prepared into a tablet, the pharmaceutical composition has good compressibility, good dissolution, and good granule flowability as compared to other pharmaceutical combination modes.

Furthermore, through the selection of various components and the control of their respective contents, a stable, pharmaceutically acceptable pharmaceutical formulation with superior dissolution, superior granule flowability, and superior friability is obtained.

Meanwhile, by maintaining the particle size of the active ingredient within a suitable range, the present disclosure avoids the impact of unsuitable particle sizes on drug dissolution, thereby preventing effects on drug bioavailability. Moreover, the control of the active ingredient's particle size in the present disclosure can also prevent inconsistencies in drug dissolution between different batches produced with the same formula process, ensuring stable drug dissolution during the formulation manufacturing process and good reproducibility between batches. Studies have found that by controlling the D₉₀ of the active ingredient to ≤50 µm, the resulting drug exhibits minimal variation between batches, stable drug dissolution, good reproducibility between batches, and good product quality.

### DETAILED DESCRIPTION

In order to make the objective, technical solutions, and advantages of the present disclosure more apparent, the present disclosure is further illustrated in detail below. It is apparent that the described examples are only a part of the examples of the present disclosure, but not all of them. All other examples obtained by those of ordinary skill in the art based on the examples in the present disclosure without making inventive efforts shall fall within the protection scope of the present disclosure.

### Examples and Comparative Examples

According to the formula ratios listed in Table 1, the active ingredient, the compound of formula (I), was jet-milled to a particle size of D90 ≤ 50 µm. The fillers, lactose monohydrate 200M, microcrystalline cellulose 101, corn starch, and mannitol 160C, were sieved. During the initial mixing, the active ingredient was first thoroughly mixed with a portion of lactose monohydrate 200M, and then, the remaining lactose monohydrate 200M, the other fillers, and low-substituted hydroxypropyl cellulose were added. The mixture was mixed uniformly. Povidone K30 was prepared into a solution with water and sprayed into the mixture for granulation. After drying in an oven, the granules were milled. During the final mixing, colloidal silicon dioxide and magnesium stearate were sieved together with the granules, and after uniform mixing, the mixture was compressed into tablets using a tablet press.

**Table 1**

| Component | | Example 1 | | Example 2 | | Comparative Example 1 | |
|---|---|---|---|---|---|---|---|
| | | Formula value/mg | Ratio/ wt% | Formula value/mg | Ratio/ wt% | Formula value/mg | Ratio/ wt% |
| Active ingredient | Compound of formula (I) | 5 | 2.5 | 5 | 2.5 | 5 | 2.5 |
| Filler | Lactose monohydrate 200M | 149 | 74.5 | 149 | 74.5 | 149 | 74.5 |
| | Microcrystalline cellulose 101 | 30 | 15 | / | / | / | / |
| | Corn starch | / | / | 30 | 15 | / | / |
| | Mannitol 160C | / | / | / | / | 30 | 15 |
| Binder | Povidone K30 | 10 | 5 | 10 | 5 | 10 | 5 |
| Disintegrant | Low-substituted hydroxypropyl cellulose | 4 | 2 | 4 | 2 | 4 | 2 |
| Glidant | Colloidal silicon dioxide | 1 | 0.5 | 1 | 0.5 | 1 | 0.5 |
| Lubricant | Magnesium stearate | 1 | 0.5 | 1 | 0.5 | 1 | 0.5 |
| Wetting agent | Water | Appropriate amount | / | Appropriate amount | / | Appropriate amount | / |
| Total | | 200.0 | 100 | 200.0 | 100 | 200.0 | 100 |

According to the formula ratios listed in Table 2, the active ingredient, the compound of formula (I), was jet-milled to a particle size of D90 ≤ 50 µm. The fillers, lactose monohydrate 200M, microcrystalline cellulose 101, and fully pregelatinized starch, were sieved. During the initial mixing, the active ingredient was thoroughly mixed with the filler lactose monohydrate (or fully pregelatinized starch), microcrystalline cellulose 101, and sodium carboxymethyl starch. Povidone K30 was prepared into a solution with water and added for granulation. After drying in an oven, the granules were milled. During the final mixing, colloidal silicon dioxide and magnesium stearate were sieved together with the granules, and after uniform mixing, the mixture was compressed into tablets using a tablet press.

**Table 2**

| Component | | Example 3 | | Comparative Example 2 | |
|---|---|---|---|---|---|
| | | Formula value/mg | Ratio/wt% | Formula value/mg | Ratio/wt% |
| Active ingredient | Compound of formula (I) | 12 | 5% | 12 | 5% |
| Filler | Fully pregelatinized starch | - | - | 139.2 | 58% |
| | Microcrystalline cellulose 101 | 74.4 | 31% | 74.4 | 31% |
| | Lactose monohydrate 200M | 139.2 | 58% | - | - |
| Disintegrant | Sodium carboxymethyl starch | 4.8 | 2% | 4.8 | 2% |
| Binder | Povidone K30 | 7.2 | 3% | 7.2 | 3% |
| Glidant | Colloidal silicon dioxide | 1.2 | 0.5% | 1.2 | 0.5% |
| Lubricant | Magnesium stearate | 1.2 | 0.5% | 1.2 | 0.5% |
| Wetting agent | Water | Appropriate amount | / | Appropriate amount | / |
| Total | | 240 | 100 | 240 | 100 |

According to the formula ratios listed in Table 3, the active ingredient, the compound of formula (I), was jet-milled to a particle size of D90 ≤ 50 µm. The fillers, lactose monohydrate 200M and microcrystalline cellulose 101, were sieved. During the initial mixing, the active ingredient was first thoroughly mixed with a portion of lactose monohydrate 200M, and then, the remaining lactose monohydrate, microcrystalline cellulose 101, and sodium carboxymethyl starch were added. The mixture was mixed uniformly. Povidone K30 was prepared into a solution with water and sprayed into the mixture for granulation. After drying in an oven, the granules were milled. During the final mixing, colloidal silicon dioxide and magnesium stearate were sieved together with the granules, and after uniform mixing, the mixture was compressed into tablets using a tablet press.

**Table 3**

| Component | | Example 4 | | Example 5 | | Example 6 | | Example 7 | |
|---|---|---|---|---|---|---|---|---|---|
| | | Formula value/mg | Ratio /wt% | Formula value/mg | Ratio /wt% | Formula value/mg | Ratio /wt% | Formula value/mg | Ratio /wt% |
| Active ingredient | Compound of formula (I) | 5 | 2.5 | 5 | 2.5 | 5 | 2.5 | 5 | 2.5 |
| Filler | Lactose monohydrate 200M | 90 | 45.0 | 60 | 30.0 | 149 | 74.5 | 134 | 67 |
| | Microcrystalline cellulose 101 | 89 | 44.5 | 119 | 59.5 | 30 | 15 | 45 | 22.5 |
| Binder | Povidone K30 | 6 | 3 | 6 | 3 | 6 | 3 | 6 | 3 |
| Disintegrant | Sodium carboxymethyl starch | 8 | 4 | 8 | 4 | 8 | 4 | 8 | 4 |
| Glidant | Colloidal silicon dioxide | 1 | 0.5 | 1 | 0.5 | 1 | 0.5 | 1 | 0.5 |
| Lubricant | Magnesium stearate | 1 | 0.5 | 1 | 0.5 | 1 | 0.5 | 1 | 0.5 |
| Wetting agent | Water | Appropriate amount | / | Appropriate amount | / | Appropriate amount | / | Appropriate amount | / |
| Total | | 200.0 | 100 | 200.0 | 100 | 200.0 | 100 | 200.0 | 100 |

According to the formula ratios listed in Table 4, the active ingredient, the compound of formula (I), was jet-milled to a particle size of D90 ≤ 50 µm. Lactose monohydrate 200M, microcrystalline cellulose 101, and hydroxypropyl cellulose EXF were sieved. During the initial mixing, the active ingredient was first thoroughly mixed with a portion of lactose monohydrate 200M, and then, the remaining lactose monohydrate 200M, microcrystalline cellulose 101, and crospovidone XL-10 were added. The mixture was mixed uniformly. When hydroxypropyl cellulose EXF was used as the binder, it was mixed in as a dry powder added internally. When povidone K30 was used as the binder, it was prepared into a solution with water and added for wet granulation. After drying in an oven, the granules were milled. During the final mixing, colloidal silicon dioxide and magnesium stearate were sieved together with the granules, and after uniform mixing, the mixture was compressed into tablets using a tablet press.

**Table 4**

| Component | | Example 8 | | Example 9 | |
|---|---|---|---|---|---|
| | | Formula value/mg | Ratio/wt% | Formula value/mg | Ratio/wt% |
| Active ingredient | Compound of formula (I) | 5 | 2.5 | 5 | 2.5 |
| Filler | Lactose monohydrate 200M | 149 | 74.5 | 149 | 74.5 |
| | Microcrystalline cellulose 101 | 30 | 15 | 30 | 15 |
| Binder | Povidone K30 | 10 | 5 | / | / |
| | Hydroxypropyl cellulose EXF | / | / | 10 | 5 |
| Disintegrant | Crospovidone XL-10 | 4 | 2 | 4 | 2 |
| Glidant | Colloidal silicon dioxide | 1 | 0.5 | 1 | 0.5 |
| Lubricant | Magnesium stearate | 1 | 0.5 | 1 | 0.5 |
| Wetting | Water | Appropriate | / | Appropriat | / |
| agent | | amount | | e amount | |
| Total | | 200.0 | 100 | 200.0 | 100 |

According to the formula ratios listed in Table 5, the active ingredient, the compound of formula (I), was jet-milled to a particle size of D90 ≤ 50 µm. Lactose monohydrate 200M and microcrystalline cellulose 101 were sieved. During the initial mixing, the active ingredient was first thoroughly mixed with a portion of lactose monohydrate 200M, and then, the remaining lactose monohydrate 200M and microcrystalline cellulose 101 were added. The mixture was mixed uniformly. The disintegrants, sodium carboxymethyl starch, low-substituted hydroxypropyl cellulose, and crospovidone XL-10, were added separately, and the mixture was mixed uniformly. Povidone K30 was prepared into a solution with water and added for wet granulation. After drying in an oven, the granules were milled. During the final mixing, colloidal silicon dioxide and magnesium stearate were sieved together with the granules, and after uniform mixing, the mixture was compressed into tablets using a tablet press.

**Table 5**

| Component | | Example 10 | | Example 11 | | Example 12 | |
|---|---|---|---|---|---|---|---|
| | | Formula value/mg | Ratio/ wt% | Formula value/mg | Ratio/ wt% | Formula value/mg | Ratio/ wt% |
| Active ingredient | Compound of formula (I) | 5 | 2.5 | 5 | 2.5 | 5 | 2.5 |
| Filler | Lactose monohydrate 200M | 149 | 74.5 | 149 | 74.5 | 149 | 74.5 |
| | Microcrystalline cellulose 101 | 30 | 15 | 30 | 15 | 30 | 15 |
| Binder | Povidone K30 | 6 | 3 | 6 | 3 | 6 | 3 |
| Disintegrant | Sodium carboxymethyl starch | 8 | 4 | / | / | / | / |
| | Low-substituted hydroxypropyl cellulose | / | / | 8 | 4 | / | / |
| | Crospovidone XL-10 | / | / | / | / | 8 | 4 |
| Glidant | Colloidal silicon dioxide | 1 | 0.5 | 1 | 0.5 | 1 | 0.5 |
| Lubricant | Magnesium stearate | 1 | 0.5 | 1 | 0.5 | 1 | 0.5 |
| Wetting agent | Water | Appropriate amount | / | Appropriate amount | / | Appropriate amount | / |
| Total | | 200.0 | 100 | 200.0 | 100 | 200.0 | 100 |

According to the formula ratios listed in Table 6, the active ingredient was jet-milled to a particle size of D90 ≤ 50 µm. The fillers, lactose monohydrate 200M and microcrystalline cellulose 101, were sieved. During the initial mixing, the active ingredient was first thoroughly mixed with a portion of lactose monohydrate 200M, and then, the remaining lactose monohydrate, microcrystalline cellulose 101, and sodium carboxymethyl cellulose were added. The mixture was mixed uniformly. Povidone K30 was prepared into a solution with water and added for wet granulation. After drying in an oven, the granules were milled. During the final mixing, colloidal silicon dioxide and/or magnesium stearate were sieved together with the granules, and after uniform mixing, the mixture was compressed into tablets using a tablet press.

**Table 6**

| Component | | Example 13 | | Comparative Example 3 | | Example 14 | | Comparative Example 4 | |
|---|---|---|---|---|---|---|---|---|---|
| | | Formula value/mg | Ratio /wt% | Formula value/mg | Ratio /wt% | Formula value/mg | Ratio /wt% | Formula value/mg | Ratio/ wt% |
| Active ingredient | Compound of formula (I) | 6 | 2.5 | 6 | 2.50 | 6 | 2.50 | 6 | 2.50 |
| Filler | Lactose monohydrate 200M | 178.08 | 74.2 | 178.08 | 74.20 | 178.08 | 74.20 | 178.08 | 74.20 |
| | Microcrystalline cellulose 101 | 36 | 15 | 36 | 15.00 | 36 | 15.00 | 36 | 15.00 |
| Binder | Povidone K30 | 7.2 | 3 | 7.2 | 3.00 | 7.2 | 3.00 | 7.2 | 3.00 |
| Disintegrant | Sodium carboxymethyl starch | 9.6 | 4 | 9.6 | 4.00 | 9.6 | 4.00 | 9.6 | 4.00 |
| Glidant | Colloidal silicon dioxide | 1.2 | 0.5 | 1.2 | 0.50 | / | / | / | / |
| Lubricant | Magnesium stearate | 1.92 | 0.8 | / | / | 1.92 | 0.80 | / | / |
| Wetting agent | Water | Appropriate amount | / | Appropriate amount | / | Appropriate amount | / | Appropriate amount | / |

### Experimental Example 1: Compressibility Comparison

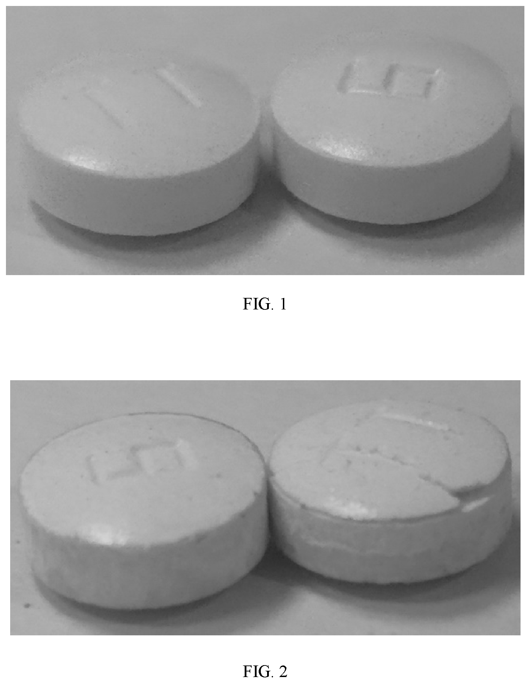

During the tablet preparation process for Examples 1-14 and Comparative Examples 1-4, the tableting process was observed to assess the compressibility. If tablets could be easily formed, could be compressed to an appropriate hardness under moderate pressure, and had a smooth and intact surface, the compressibility was considered good, as shown in FIG. 1. If problems such as capping, lamination, sticking, or picking occurred during tableting, the compressibility was considered poor. The results are shown in Table 7:

**Table 7**

| Investigation item | Examples 1-14 | Comparative Example 1 | Comparative Example 2 | Comparative Example 3 | Comparative Example 4 |
|---|---|---|---|---|---|
| Compressibility | Good | Picking | Cannot be formed | Sticking | Sticking |

Sticking: refers to the phenomenon where fine powder from the tablet surface adheres to the punches and dies during tableting, resulting in a rough, uneven, or pitted tablet surface, as shown in FIG. 2.

Picking: refers to the abnormal movement of the upper and lower punches during the operation of the tablet press.

### Experimental Example 2: Dissolution Comparison

Dissolution experiment: The dissolution of tablets from Examples 1-13 was determined according to the Dissolution and Drug Release Test Method (General Rule 0931 Method 2, Chinese Pharmacopoeia, 2015 Edition, Part IV; USP<711>). 900 mL of a dissolution medium was used (7.8 g of sodium dihydrogen phosphate dihydrate was taken, dissolved in water and diluted to 1000 mL, and adjusted to pH 4.50 ± 0.05 using a 1 mol/L sodium hydroxide solution or 10% dilute phosphoric acid, and 0.5 g of sodium lauryl sulfate was added and stirred to dissolve). The dissolution experiment was conducted with a paddle speed of 50 rpm at 37.0 °C ± 0.5 °C.

A comparison of dissolution under storage conditions (0 days) is shown in Table 8:

**Table 8**

| Example | Dissolution/% | | | | | |
|---|---|---|---|---|---|---|
| | 5 min | 10 min | 15 min | 30 min | 45 min | 60 min |
| Example 1 | 38.6 | 64.3 | 76.1 | 88.2 | 92.0 | 94.1 |
| Example 2 | 25.0 | 48.0 | 62.5 | 79.8 | 84.6 | 85.2 |
| Example 3 | 45.7 | 64.9 | 74.5 | 87 | 91.4 | 94.1 |
| Example 4 | 52.5 | 69.6 | 77.7 | 88.7 | 92.4 | 93.8 |
| Example 5 | 51.1 | 67.6 | 76.0 | 87.0 | 90.7 | 92.8 |
| Example 6 | 49.6 | 69.1 | 76.9 | 84.1 | 85.6 | 86.0 |
| Example 7 | 53.3 | 71.4 | 78.9 | 86.0 | 88.1 | 88.0 |
| Example 8 | 41.2 | 63.5 | 75.4 | 87.3 | 91.6 | 93.7 |
| Example 9 | 23.0 | 46.2 | 61.7 | 79.4 | 86.0 | 88.6 |
| Example 10 | 49.7 | 69.2 | 77.7 | 87.0 | 89.7 | 90.9 |
| Example 11 | 47.8 | 68.8 | 78.4 | 89.8 | 92.8 | 94.3 |
| Example 12 | 37.2 | 59.0 | 71.5 | 84.7 | 87.8 | 89.9 |
| Example 13 | 51.2 | 73.8 | 82.8 | 91.9 | 95.5 | 95.6 |

A comparison of dissolution under storage conditions (60 °C, 10 days) is shown in Table 9:

**Table 9**

| Example | Dissolution/% | | | | | |
|---|---|---|---|---|---|---|
| | 5 min | 10 min | 15 min | 30 min | 45 min | 60 min |
| Example 6 | 39.6 | 60.7 | 69.9 | 80.5 | 84.2 | 86.1 |
| Example 7 | 42.0 | 60.5 | 70.0 | 81.3 | 85.0 | 86.2 |

As can be seen from the dissolution results, the tablets from Examples 1-13 of the present disclosure under storage conditions (0 days) dissolved rapidly, reaching 80% dissolution or higher within 45 min. Meanwhile, the tablets of the present disclosure also maintained good dissolution at 60 °C for 10 days.

### Experimental Example 3: Test of Angle of Repose and Carr's Index

The Carr's index and angle of repose were measured by a powder comprehensive characteristics tester.

Angle of repose: the maximum angle formed between the free inclined surface of a powder piled layer and the horizontal plane under a static equilibrium state.

Carr's index: 1. bulk density: the density measured when powder is filled into a measuring container without applying any external force is the bulk density ρ0; 2. tapped density: a certain external force is applied, and the powder is tapped to densify the freely piled material; the tapped volume changes with the number of taps until it no longer changes, indicating the material has reached its most compact state, giving the tapped density ρf; 3. Carr's index: C = (ρf - ρ0)/ρf.

The test results are shown in Table 10:

**Table 10**

| Investigation item | Example 4 | Example 5 | Example 6 | Example 7 | Example 10 | Example 11 | Example 12 |
|---|---|---|---|---|---|---|---|
| Carr's index (%) | 17.39 | 15.91 | 17.02 | 20.41 | 18.87 | 22.22 | 24.07 |
| Angle of repose | 38° | 37° | 38° | 40° | 37° | 38° | 39° |

As can be seen from the results in Table 10, the tablets of the present disclosure have good granule flowability.

### Examples 15-1 to 15-8

According to the formula ratios of Example 15 in Table 11, the active ingredient, the compound of formula (I), was first prepared to have the particle sizes listed in Table 12 below. Then, according to the following steps, lactose monohydrate 200M and microcrystalline cellulose 101 were sieved. During the initial mixing, the compound of formula (I) was first thoroughly mixed with a portion of lactose monohydrate 200M, and then, the remaining lactose monohydrate 200M, microcrystalline cellulose 101, and sodium carboxymethyl starch were added. The mixture was mixed uniformly. Povidone K30 was prepared into a solution with water and added for wet granulation. After drying in an oven, the granules were milled. During the final mixing, colloidal silicon dioxide and magnesium stearate were sieved together with the granules, and after uniform mixing, the mixture was compressed into tablets using a tablet press.

**Table 11**

| Component | | Example 15 | |
|---|---|---|---|
| | | Formula/mg | Ratio/wt% |
| Active ingredient | Compound of formula (I) | 4 | 2.5 |
| Filler | Lactose monohydrate 200M | 118.72 | 74.2 |
| | Microcrystalline cellulose 101 | 24 | 15 |
| Binder | Povidone K30 | 4.8 | 3 |
| Disintegrant | Sodium carboxymethyl starch | 6.4 | 4 |
| Glidant | Colloidal silicon dioxide | 0.8 | 0.5 |
| Lubricant | Magnesium stearate | 1.28 | 0.8 |
| Wetting agent | Water | Appropriate amount | / |
| Total | | 200 | 100 |

**Table 12**

| Compound of formula (I) | Example 15-1 | Exampl e 15-2 | Example 15-3 | Example 15-4 | Example 15-5 | Example 15-6 | Example 15-7 | Example 15-8 |
|---|---|---|---|---|---|---|---|---|
| Particle size D90/µm | 28.9 | 23 | 18.1 | 21.1 | 34.6 | 39.8 | 50.4 | 59.7 |

### Dissolution experiment

The dissolution of tablets from Examples 15-1 to 15-8 was determined according to the Dissolution and Drug Release Test Method (General Rule 0931 Method 2, Chinese Pharmacopoeia, 2015 Edition, Part IV; USP<711>). 900 mL of a dissolution medium was used (7.8 g of sodium dihydrogen phosphate dihydrate was taken, dissolved in water and diluted to 1000 mL, and adjusted to pH 4.50 ± 0.05 using a 1 mol/L sodium hydroxide solution or 10% dilute phosphoric acid, and 1.0 g of sodium lauryl sulfate was added and stirred to dissolve). The dissolution experiment was conducted with a paddle speed of 75 rpm at 37.0 °C ± 0.5 °C.

**Table 12**

| Example | Dissolution/%^{*2} | | | | | | Similarity factor *f*₂ |
|---|---|---|---|---|---|---|---|
| | 5 min | 10 min | 15 min | 30 min | 45 min | 60 min | |
| Example 15-1 | 63.6 | 86.5 | 91.3 | 95.3 | 96.4 | 97.0 | Reference |
| Example 15-2 | 71 | 89 | 94 | 98 | 98 | 98 | 71.2 |
| Example 15-3 | 74.6 | 93.0 | 96.6 | 99.1 | 99.3 | 99.6 | 60.6 |
| Example 15-4 | 71.8 | 87.8 | 92.3 | 95.5 | 96.7 | 96.8 | 72.4 |
| Example 15-5 | 58.6 | 76.2 | 82.5 | 89.6 | 91.7 | 92.6 | 58.0 |
| Example 15-6 | 62.0 | 75.8 | 81.2 | 87.8 | 90.1 | 91.4 | 55.8 |
| Example 15-7 | 67.7 | 81.7 | 87.2 | 93.9 | 95.3 | 96.0 | 73.8 |
| Example 15-8 | 42.0 | 57.2 | 65.6 | 77.2 | 82.8 | 85.9 | 34.0 |

The results indicate that the tablets from Examples 15-2 to 15-7 exhibit a relatively fast dissolution rate for the compound of formula (I), reaching 90% dissolution or higher within 45 min, and the dissolution similarity factor f2 was greater than 50, demonstrating substantially consistent dissolution between batches. In contrast, the tablets from Example 15-8 exhibit a relatively slower dissolution rate, with only 82.8% dissolution within 45 min and a similarity factor f2 of less than 50, indicating inconsistent dissolution between batches.

Friability: reference was made to Friability Test for Tablets (General Rule 0923, Chinese Pharmacopoeia, 2015 Edition, Part IV).

The above description is only for the purpose of illustrating preferred examples of the present disclosure, and is not intended to limit the scope of the present disclosure. Any modifications, equivalent substitutions, improvements, and the like made without departing from the spirit and principle of the present disclosure should be included in the protection scope of the present disclosure.

## Claims

1. A pharmaceutical composition, comprising 1 wt%-70 wt% of an active ingredient, wherein the active ingredient is a compound of formula (I) or a pharmaceutically acceptable salt, crystal form, or isomer thereof, and at least one pharmaceutical excipient;

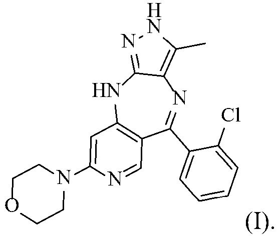

2. The pharmaceutical composition according to claim 1, wherein the pharmaceutical excipient comprises a filler, a binder, a disintegrant, or a lubricant.

3. The pharmaceutical composition according to claim 2, wherein the pharmaceutical excipient further comprises a glidant.

4. The pharmaceutical composition according to claim 3,
comprising 1 wt%-30 wt% of the compound of formula (I) or the pharmaceutically acceptable salt, crystal form, or isomer thereof; 10 wt%-90 wt% of the filler; 0.1 wt%-10 wt% of the binder; 1 wt%-10 wt% of the disintegrant; 0.1 wt%-2 wt% of the lubricant; and 0.1 wt%-2 wt% of the glidant;
preferably comprising 1 wt%-10 wt% of the compound of formula (I) or the pharmaceutically acceptable salt, crystal form, or isomer thereof; 80 wt%-90 wt% of the filler; 2 wt%-5 wt% of the binder; 2 wt%-8 wt% of the disintegrant; 0.6 wt%-1 wt% of the lubricant; and 0.5 wt%-1 wt% of the glidant.

5. The pharmaceutical composition according to claim 3, wherein the filler is selected from: one or more of lactose monohydrate, microcrystalline cellulose, corn starch, glucose, mannitol, sorbitol, calcium carbonate, and calcium hydrogen phosphate; preferably, the filler is selected from lactose monohydrate and microcrystalline cellulose.

6. The pharmaceutical composition according to claim 3, wherein the binder is selected from: one or more of povidone, hydroxypropyl cellulose, methyl cellulose, hydroxypropyl methylcellulose, and sodium carboxymethyl cellulose; preferably, the binder is selected from povidone.

7. The pharmaceutical composition according to claim 3, wherein the disintegrant is selected from: one or more of sodium carboxymethyl starch, low-substituted hydroxypropyl cellulose, crospovidone, and croscarmellose sodium; preferably, the disintegrant is selected from sodium carboxymethyl starch.

8. The pharmaceutical composition according to claim 3, wherein the lubricant is selected from: magnesium stearate and sodium stearyl fumarate; the glidant is selected from: colloidal silicon dioxide and talcum powder.

9. The pharmaceutical composition according to claim 1, wherein a particle size distribution range of the compound of formula (I) or the pharmaceutically acceptable salt, crystal form, or isomer thereof is D90 < 60 µm; preferably, D90 ≤ 58 µm.

10. The pharmaceutical composition according to claim 1, wherein a particle size distribution range of the compound of formula (I) or the pharmaceutically acceptable salt, crystal form, or isomer thereof is D90 ≤ 50 µm.

11. The pharmaceutical composition according to any one of claims 1-10, wherein a content of the compound of formula (I) or the pharmaceutically acceptable salt, crystal form, or isomer thereof is 1-20 mg.

12. A pharmaceutical formulation, wherein the pharmaceutical formulation is prepared from the pharmaceutical composition according to any one of claims 1-11.

13. The pharmaceutical formulation according to claim 12, wherein the pharmaceutical formulation is a tablet.

14. A kit, comprising the pharmaceutical formulation according to claim 12 and instructions for use.

15. Use of the pharmaceutical composition according to any one of claims 1-11 or the pharmaceutical formulation according to claim 12 in the manufacture of a medicament for treating a cancer.

16. The use according to claim 15, wherein the cancer comprises cholangiocarcinoma, head and neck cancer, liver cancer, colorectal cancer, osteosarcoma, bladder cancer, prostate cancer, gastric cancer, urothelial carcinoma, lung cancer, endometrial cancer, breast cancer, thyroid cancer, ovarian cancer, peritoneal cancer, renal cancer, B-cell malignancy, oral cancer, and nasopharyngeal carcinoma.

17. A preparation method for the pharmaceutical composition according to any one of claims 1-11 or the pharmaceutical formulation according to claim 12 or 13, wherein the preparation is performed by a wet granulation method.
